(19) Europäisches Patentamt
European Patent Office
Office européen des brevets

(11) Publication number : **0 523 941 A1**

(12) **EUROPEAN PATENT APPLICATION**

(21) Application number : **92306404.2**

(22) Date of filing : **13.07.92**

(51) Int. Cl.[5] : **C07D 403/12,** A61K 31/415

(30) Priority : **11.07.91 GB 9115005**

(43) Date of publication of application :
**20.01.93 Bulletin 93/03**

(84) Designated Contracting States :
**PT**

(71) Applicant : **RHONE-POULENC RORER LIMITED**
**RPR House, St. Leonards Road**
**Eastbourne, East Sussex BN21 3YG (GB)**

(72) Inventor : **Pitchen, Phillipe**
**c/o Rhone-Poulenc Rorer Limited, Health Care Div.**
**Dagenham, Essex RM10 7XS (GB)**

(74) Representative : **Bentham, Stephen et al**
**J.A. Kemp & Co. 14 South Square Gray's Inn**
**London, WC1R 5LX (GB)**

(54) **Imidazoles as ACAT inhibitor.**

(57) The enantiomer of 2-[5-(3,5-dimethylpyrazol-1-yl)pentylsulphinyl]-4,5-diphenylimidazole which, in dilute solution in dichloromethane at room temperature, rotates plane-polarised light of the sodium D wavelength to the right, or a pharmaceutically acceptable acid addition salt thereof which possesses useful pharmacological properties.

EP 0 523 941 A1

Jouve, 18, rue Saint-Denis, 75001 PARIS

This invention relates to new therapeutically useful imidazole derivatives, to processes for their preparation and to pharmaceutical compositions containing them.

In the specification of PCT Patent Application No. EP 91/00023, Publication No. WO 91/10662, as well as in the specifications of the corresponding patent applications in other countries claiming priority from British Patent Applications Nos. 9000696.6 and 9027892.0, there are discussed compounds of formula I:-

$$[DPIM]\text{-}S(O)_p\text{-}W\text{-}Y \qquad I$$

wherein [DPIM]is as hereinafter depicted, wherein R and $R^1$ are the same or different and each represents a hydrogen or halogen atom or a straight- or branched-chain alkyl or alkoxy group containing from 1 to about 6 carbon atoms, p represents 0, 1 or 2, W represents a methylene group or an alkylene chain of 2 to about 6 carbon atoms optionally substituted with one or more straight- or branched-chain alkyl groups of 1 to about 4 carbon atoms, and Y represents a 5- or 6-membered unsaturated ring containing 1, 2, 3 or 4 nitrogen atoms, optionally substituted by one or more substituents selected from straight- and branched-chain alkyl groups containing from 1 to about 6 carbon atoms (which are each optionally substituted by a phenyl group), phenyl groups, amino groups and nitro groups, and pharmaceutically acceptable acid addition salts thereof.

In the aforesaid specifications it is stated that the said compounds have valuable pharmacological properties, in particular properties which are indicative of utility in the treatment of conditions which can be ameliorated by the administration of an inhibitor of acyl coenzyme-A:cholesterol-O-acyl transferase (ACAT; EC 2.3.1.26), such as atherosclerosis, hyperlipidaemia, cholesterol ester storage disease and atheroma in vein grafts.

In the aforementioned specifications, reference is made to the optical activity of the compounds of formula I wherein p represents 1.

As a result of further extensive research and experimentation, it has now been found, surprisingly, that one of the enantiomeric forms of the compound of formula I wherein R and $R^1$ each represent hydrogen atoms, p represents 1, W represents a pentamethylene group, and Y represents a 3,5-dimethylpyrazol-1-yl group, hereinafter referred to as "enantiomer A", possesses properties which are indicative of outstanding utility in the treatment of one or more of such disorders. The enantiomer of the invention presents, in some aspects of its activities, an unexpected improvement over the other compounds of the aforementioned specifications.

Its beneficial properties are enhanced by its relatively low mammalian toxicity.

Thus, the present invention provides one of the enantiomeric forms, i.e. "enantiomer A", of the compound 2-[5-(3,5-dimethylpyrazol-1-yl)pentylsulphinyl]-4,5-diphenylimidazole of formula II, hereinafter depicted, and pharmaceutically acceptable acid addition salts thereof.

It is thought that the said enantiomer A is actually the (S)-enantiomer, i.e. (S)-2-[5-(3,5-dimethylpyrazol-1-yl)pentylsulphinyl]-4,5-diphenylimidazole and, as such, it provides for further features of the invention.

Although enantiomer A falls within the scope of general formula I, and in the aforementioned specifications there is described the preparation and properties of its 1:1 mixture with its less desirable enantiomer (hereinafter referred to as "enantiomer B"), enantiomer A has not been specifically described or characterised hitherto.

Enantiomer A is distinguished from enantiomer B by the different directions of their optical rotations in various solvents, and by their different retention times on chiral chromatography absorbents, in common with other pairs of enantiomers, but, much more importantly, enantiomer A is distinguished from enantiomer B by their markedly different biological activities.

In dilute solution in dichloromethane at room temperature enantiomer A rotates plane-polarised light of the sodium D wavelength to the right, whilst enantiomer B rotates it to the left.

However, in dilute solution in methanol at room temperature enantiomer A rotates plane-polarised light of the sodium D wavelength to the left, whilst enantiomer B rotates it to the right.

When subjected to HPLC on cellulose tris(3,5-dimethylphenylcarbamate) on a silica gel support, eluting with a mixture of methanol, isopropanol and n-heptane (5:2:93v/v), enantiomer A has a longer retention time than enantiomer B.

Enantiomer A and enantiomer B have been subjected to a variety of laboratory tests, which are believed to correlate to pharmacological activity in humans and other animals.

Surprisingly, enantiomer A is at least ten times more active in the inhibition of rat liver ACAT and rabbit tissue ACAT than enantiomer B, but whereas intravenous administration of enantiomer B causes emesis in dogs, after only 5 minutes, enantiomer A is remarkably free from that disadvantageous side-effect.

In assays performed in-vitro, microsomes, obtained from the livers of rats fed on a diet supplemented with 0.5%w/w cholesterol and 0.25%w/w cholic acid for 7 days, were incubated with radiolabelled oleoyl-CoA in the presence of enantiomer A or of enantiomer B at various concentrations. The degree of ACAT inhibition produced in each test was measured and the $IC_{50}$ figures for each enantiomer were calculated and are shown in Table 1.

Corresponding tests were performed using tissue from rabbits instead of rats' livers, and omitting the cholic

acid supplement from their diet, and the two $IC_{50}$ figures calculated are shown in Table I.

Table I

| Compound | $IC_{50}$ Rat liver ACAT | $IC_{50}$ Rabbit tissue ACAT |
|---|---|---|
| Enantiomer A | 111nM | 122nM |
| Enantiomer B | >1000nM | 1453nM |

The present invention also provides a method for the treatment of a human or animal patient suffering from, or subject to, conditions which can be ameliorated by the administration of an inhibitor of acyl coenzyme-A:cholesterol-O-acyl transferase, such as the treatment of atherosclerosis, hyperlipidaemia, cholesterol ester storage disease, atheroma in vein grafts, and the reduction of atherosclerotic plaque, which comprises administering to the patient an effective amount of enantiomer A, or a pharmaceutically acceptable acid addition salt thereof, as hereinbefore defined, to secure an improvement in the condition of the patient.

The invention also provides enantiomer A, or a pharmaceutically acceptable acid addition salt thereof, as hereinbefore defined, for use in a new method of treatment, of the human or animal body, by therapy, of conditions which can be ameliorated by the administration of an inhibitor of acyl coenzyme-A:cholesterol-O-acyl transferase, such as atherosclerosis, hyperlipidaemia, cholesterol ester storage disease and atheroma in vein grafts, or the reduction of atherosclerotic plaque.

Enantiomer A may be prepared by the application or adaptation of known methods, for example methods described in the aforementioned specifications for the preparation of compounds of formula I.

Thus, according to features of the present invention, enantiomer A, free or virtually free from enantiomer B, is obtained by separation of the enantiomers arising from a non-selective oxidation or by using known enantio-selective oxidising systems.

According to one feature of the present invention, a mixture of enantiomers of 2-[5-(3,5-dimethylpyrazol-1-yl)pentylsulphinyl]-4,5-diphenylimidazole of formula II, e.g. the racemic mixture, is subjected to chromatography, e.g. preparative HPLC using a chiral adsorbent, for example cellulose tris(3,5-dimethylphenylcarbamate) on a silica gel support (for example as commercialised under the name "Chiralcel OD"), eluting with a suitable solvent system, for example a mixture of methanol, isopropanol and n-heptane, for example in a ratio of about 5:2:93v/v, to produce enantiomer A, free or virtually free from enantiomer B.

As explained in the aforementioned specifications, racemic 2-[5-(3,5-dimethylpyrazol-1-yl)pentylsulphinyl]-4,5-diphenylimidazole of formula II may be prepared by the oxidation of 2-[5-(3,5-dimethylpyrazol-1-yl)pentylthio]-4,5-diphenylimidazole of formula III, hereinafter depicted, the oxidation being conveniently carried out by using a conventional oxidant, such as hydrogen peroxide, sodium metaperiodate, a hypochlorite, an acyl nitrite, sodium perborate, peracids, such as percarboxylic acids e.g. m-chloroperbenzoic acid), potassium permanganate or potassium hydrogen persulphate, or a ruthenium VIII compound, in an inert solvent, at or below room temperature.

2-[5-(3,5-Dimethylpyrazol-1-yl)pentylthio]-4,5-diphenylimidazole of formula III can be prepared by the reaction of 4,5-diphenylimidazole-thiol of formula IV, hereinafter depicted, or a salt thereof, with a compound of general formula V, hereinafter depicted, or a salt thereof, wherein $X^1$ represents a group displaceable by a thiolate salt, such as a halogen e.g. a chlorine, bromine or iodine, atom or an alkyl- or aryl- sulphonyloxy group (e.g. methanesulphonyloxy or 4-toluenesulphonyloxy). The reaction is generally carried out in an inert organic solvent such as tetrahydrofuran or dimethylformamide at a temperature from ambient to 110°C and optionally in the presence of a proton acceptor, such as an amine (e.g. triethylamine).

According to a further feature of the present invention, enantiomer A, free or virtually free from enantiomer B, is prepared from the appropriate enantiomer of a compound of the general formula VI, hereinafter depicted, wherein Y represents a protective group, for example a trialkylsilylalkoxymethyl group, e.g. a trialkylsilylethoxymethyl group, preferably a trimethylsilylethoxymethyl group, or an optionally substituted benzyl group, such as an alkoxy- or dialkoxy-benzyl group, for example a dimethoxybenzyl group, e.g. a 3,4-dimethoxybenzyl, 2,4-dimethoxybenzyl or 3,5-dimethoxybenzyl group, or a methoxybenzyl group, e.g. a 4-methoxybenzyl group, by the replacement of the said protective group by a hydrogen atom by the application or adaptation of known methods. The reaction can be carried out by hydrolysis, for example by using a dilute acid, e.g. hydrochloric acid, or by using a mixture of trifluoroacetic acid and anisole, or alternatively by reaction with tetrabutylammonium fluoride, preferably in an ethereal medium, e.g. tetrahydrofuran.

The appropriate enantiomer of the compound of the general formula VI, hereinbefore defined, free or virtually free from the undesired enantiomer, can be prepared by the reaction of the appropriate enantiomer of a compound of general formula VII, hereinafter depicted, wherein Y is as hereinbefore defined, and $X^2$ represents a halogen, preferably chlorine, atom, with 3,5-dimethylpyrazole, or a salt thereof, by the application or adaptation of conditions similar to those hereinbefore described for the reaction of compounds of formula IV with compounds with compounds of formula V.

The appropriate enantiomer of the compound of the general formula VII, hereinbefore defined, free or virtually free from the undesired enantiomer, can be prepared by the reaction of the appropriate enantiomer of a compound of general formula VIII, hereinafter depicted, wherein Y is as hereinbefore defined with a compound of the general formula:-

$$X^3\text{-}(CH_2)_4\text{-}X^2 \qquad IX$$

wherein $X^2$ is as hereinbefore defined, and $X^3$ represents a halogen, preferably iodine, atom, or a salt thereof, in the presence of a strong base, such as lithium diisopropylamide, and in the presence of a mixture of solvents such as tetrahydrofuran and 1,3-dimethylimidazolidinone or hexamethylphosphoramide.

According to a further feature of the present invention, the appropriate enantiomer of the compound of the general formula VIII, hereinbefore defined, free or virtually free from the undesired enantiomer, is prepared by the enantio-selective oxidation of a compound of general formula X, hereinafter depicted, wherein Y is as hereinbefore defined.

The enantio-selective oxidation may be carried out by the application or adaptation of known enantio-selective oxidising systems, for example a mixture of diethyl D-tartrate, titanium isopropoxide and an organic hydroperoxide, e.g. tert-butyl hydroperoxide or, preferably, cumene hydroperoxide, in a suitable solvent such as dichloromethane, preferably under an inert atmosphere.

Compounds of general formula X may be prepared by the application or adaptation of known methods, for example methods described in the following Reference Examples.

Alternatively, the appropriate enantiomer of a compound of the general formula VI, hereinbefore defined, free or virtually free from the undesired enantiomer, can be prepared from its racemic form, by preparative HPLC using a chiral adsorbent, in conditions similar to those described hereinbefore for the resolution of the enantiomers of formula II.

The racemic compounds of formula VI can be prepared by known methods, for example by proceeding in a similar manner to that described hereinbefore for the separate enantiomers of formula VI but using a known non-enantio-selective oxidising system for the preparation of the racemic precursor of formula VIII, or by replacement by a protecting group Y of the hydrogen atom in the N-H moiety in a mixture of enantiomers A and B of formula II, by the application or adaptation of known methods, e.g. by reaction with a compound of the general formula:-

$$Y\text{-}X^2 \qquad IX$$

wherein $X^2$ and Y are as hereinbefore defined.

According to yet a further feature of the invention, enantiomer A of 2-[5-(3,5-dimethylpyrazol-1-yl)-pentylsulphinyl]-4,5-diphenylimidazole of formula II is prepared by the oxidation of 2-[5-(3,5-dimethylpyrazol-1-yl)pentylthio]-4,5-diphenylimidazole of formula III using an enantio-selective oxidising system. A particularly effective example of such a system involves the use of a chiral oxaziridine oxidising agent. Such agents are well known in the art, and a particularly suitable agent is the so-called "(+)-8,8-dichlorocamphorylsulfonyloxaziridine", whose structure is depicted hereinafter, and which is an item of commerce. Its preparation and use are described, for example, by Davis et al., in their papers such as J. Org. Chem., (1990), 55, 3715-3717 .

By the term "known methods" as used in this specification is meant methods used heretofore or described in the literature.

By the term "pharmaceutically acceptable salts" as used in this specification is meant acid addition salts the anions of which are relatively innocuous to the animal organism when used in therapeutic doses so the the beneficial pharmaceutical properties of enantiomer A are not vitiated by side-effects ascribable to those anions.

It is to be understood that, where in this specification reference is made to enantiomer A, it is intended to refer also, where the context so permits, to its pharmaceutically acceptable salts.

Suitable acid addition salts for use in pharmaceuticals may by selected from salts derived from inorganic acids, for example hydrochlorides, hydrobromides, phosphates, sulphates and nitrates, and organic acids, for example oxalates, lactates, tartrates, acetates, salicylates, citrates, propionates, succinates, fumarates, maleates, methylene-bis-β-hydroxynaphthoates, gentisates and di-p-toluoyltartrates.

As well as being useful in themselves as active compounds, salts of enantiomer A are useful for the purposes of purification of the parent enantiomer A, for example by exploitation of the solubility differences between the salts and the parent compound, by techniques well known to those skilled in the art.

Enantiomer A can be purified by the usual physical means, for example by crystallisation or chromatography.

It was deduced that the said enantiomer A of 2-[5-(3,5-dimethylpyrazol-1-yl)pentylsulphinyl]-4,5-diphenylimidazole is probably the (S)-enantiomer, i.e. (S)-2-[5-(3,5-dimethylpyrazol-1-yl)pentylsulphinyl]-4,5-diphenylimidazole by X-ray crystallography of a sample of 2-methylsulphinyl-4,5-diphenyl-1-(4-bromobenzyl)imidazole. This sample was prepared from the compound of formula X prepared in Reference Example 6 and used in the reaction sequence which led to the formation of enantiomer A described in Reference Examples 7 and 8 and Example 7. Therefore, on the assumption that no unexpected inversion of chirality occurred during the said reaction sequence, it follows that enantiomer A has the same chirality as the said sample examined by X-ray crystallography, i.e. it is the (S)-form.

The said sample for examination by X-ray crystallography was prepared from the compound of formula X by (i) replacement of the protecting group Y by a hydrogen atom, using a method as described hereinbefore for the conversion of compounds of formula VI to enantiomer A; (ii) replacement of the said hydrogen atom by a 4-bromobenzyl group by reaction with 4-bromobenzyl bromide; and (iii) recrystallising to enantiomeric excess of 99.3% The bromobenzyl protecting group was selected because it was much more susceptible to X-ray crystallography than the 4-methoxybenzyl group, by-reason of its bromine atom.

The following Examples illustrate the invention.

Nuclear magnetic resonance (NMR) spectra were recorded at 200MHz or 400MHz. Chemical shifts are expressed in ppm relative to tetramethylsilane. Abbreviations have the following significances:-

s = singlet, d = doublet, t = triplet, q = quartet, m = multiplet, dd = doublet of doublets, etc.

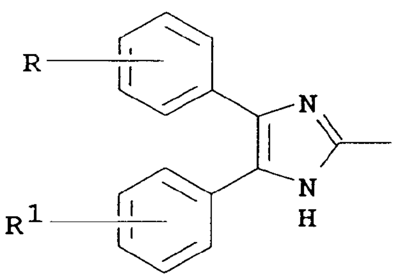

[DPIM]

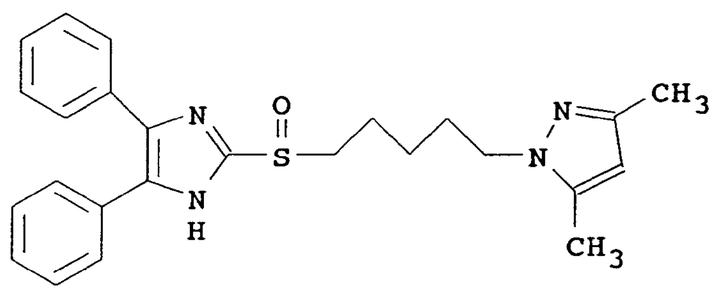

II

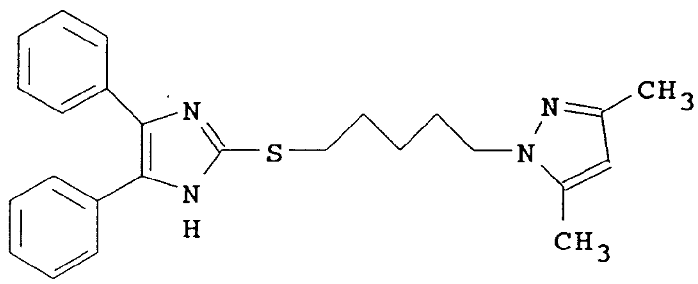

III

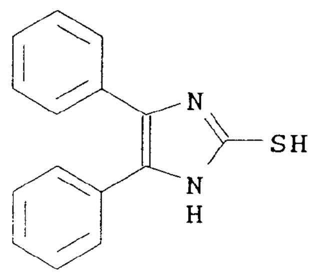

IV

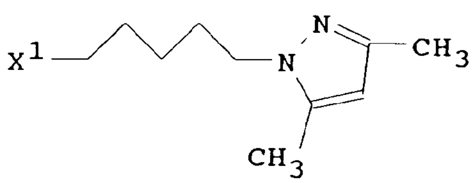

V

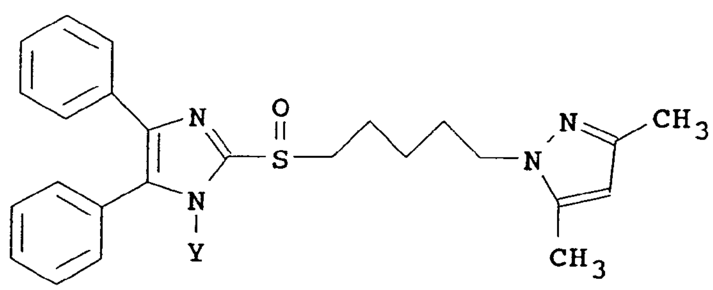

VI

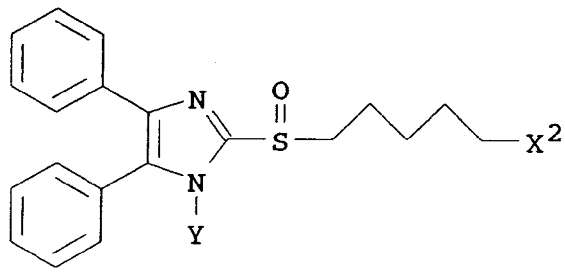

VII

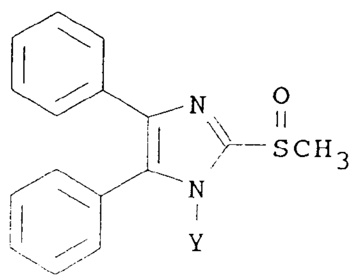

VIII

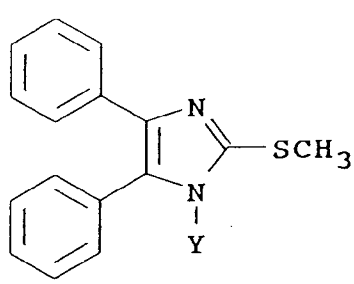

X

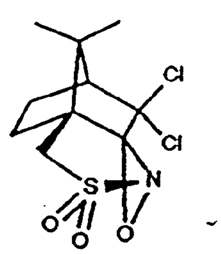

"(+)-8,8-dichlorocamphorylsulfonyloxaziridine"

EXAMPLE 1

A stirred solution of 2-[5-(3,5-dimethylpyrazol-1-yl)pentylthio]-4,5-diphenylimidazole (5.0g) in dichloromethane (250ml), cooled with in an acetone/ice bath (internal temperature -10 to -5°C) was treated with sodium hydrogen carbonate (2.0g), followed by 3-chloroperoxybenzoic acid (50-60%; 3.4g). After 35 minutes stirring in the cooling bath, the mixture was treated with sodium bicarbonate (5.0g) and dichloromethane (200ml). The organic layer was washed successively with saturated aqueous sodium metabisulphite solution (4x100ml), saturated aqueous sodium bicarbonate solution (100ml) and water (2x100ml), dried over magnesium sulphate and evaporated, to give a dark oil, which was subjected to flash chromatography on silica gel, eluting with a mixture of ethyl acetate and methanol (9:1v/v), to give 2-[5-(3,5-dimethylpyrazol-1-yl)pentylsulphinyl]-4,5-diphenylimidazole (1.2g) as an amorphous solid, m.p. 38-40°C.

EXAMPLE 2

A solution of diethyl D-tartrate (24.72g) in dry dichloromethane (430ml) stirred at room temperature under nitrogen was treated successively with titanium isopropoxide (17.04g) and distilled water (1ml). After 15 minutes stirring, a solution of 2-methylthio-4,5-diphenyl-1-[2-(trimethylsilyl)ethoxymethyl]imidazole (23.76g) in dry dichloromethane (50ml) was added and the mixture was cooled to -20°C. It was then treated with cumene hydroperoxide (22.8g; 80%) at -20°C during 10 minutes and the solution was stirred under nitrogen for 24 hours at -18 to -20C. It was then treated with distilled water (22ml) and the mixture was vigorously stirred for 1 hour at room temperature. The gel obtained was filtered and washed with dichloromethane (4x300ml). The combined filtrates were stirred for 1 hour with dilute aqueous sodium hydroxide solution (500ml;2N), washed with brine (500ml), dried over magnesium sulphate, and evaporated to dryness. The residue was subjected to flash chromatography on silica gel, eluting with a mixture of cyclohexane and ethyl acetate (7:3v/v), to give an oil, which was crystallised from pentane to give the desired enantiomer of 2-methylsulphinyl-4,5-diphenyl-1-[2-(trimethylsilyl)ethoxymethyl]imidazole (16.8g) in the form of a white solid, m.p. 67-70°C.
[Enantiomeric excess: 91%, determined by chiral HPLC (column: Chiralcel OD; mobile phase: isopropanol: heptane 4:96; UV detection at 270nm)
$[\alpha]_D^{22}$ = +3.7° (c 1.09; dichloromethane)
NMR ($CDCl_3$):- 0.00 (s,9H); 0.90 (t, 2H); 3.30 (s, 3H); 3.52 (dd,2H); 5.47 (dd, 2H); 7.20-7.22 (m, 10H)].

EXAMPLE 3

To a solution of 16g of 2-methylsulphinyl-4,5-diphenyl-1-[2-(trimethylsilyl)ethoxymethyl]imidazole (as obtained in Example 2) in 200ml of dry tetrahydrofuran stirred at -70°C under nitrogen were successively added 20ml hexamethylphosphoramide and, during 10 minutes, 21.32ml of a 2M solution of lithium diisopropylamide in heptane and tetrahydrofuran. The temperature was allowed to warm up to -25°C and a solution of 11g of 5-chloro-1-iodobutane in 30ml dry tetrahydrofuran was added over 3 minutes at -20 to -25°C. The yellow solution was stirred 15 minutes at -20°C and warmed up to room temperature during 1 hour. 100ml of a 5% aqueous solution of ammonium chloride were added to the solution and the mixture was extracted with diethyl ether (3x150ml). The combined extracts were washed with 100ml of 2N hydrochloric acid (twice), 100ml water, 100ml bring, dried over magnesium sulphate and evaporated to dryness. The residue was subjected to flash chromatography on silica gel, eluting with a mixture of cyclohexane and ethyl acetate (75:25), to give the desired enantiomer of 2-(5-chloropentylsulphinyl)-4,5-diphenyl-1-[2-(trimethylsilyl)ethoxymethyl]imidazole (17g), in the form of an oil.
[NMR ($CDCl_3$):- 0.00 (s,9H); 0.90 (dd,2H); 1.65-2.05 (m,6H); 3.40-3.73 (dm,2H); 3.49 (dd,2H); 3.59 (t,2H); 5.46 (dd,2H); 7.19-7.55 (m,10H)].

EXAMPLE 4

A solution of sodium hydride (1.86g of a 60% dispersion in mineral oil) in 70ml of dry dimethylformamide stirred under nitrogen was treated with a suspension of 5.16g of 3,5-dimethylpyrazole in 100ml of dry dimethylformamide during 30 minutes at a temperature maintained below 30°C. After stirring at room temperature for 30 minutes, 7g of sodium iodide was added. A solution of 17g of 2-(5-chloropentylsulphinyl)-4,5-diphenyl-1-[2-(trimethylsilyl)ethoxymethyl]imidazole (as obtained in Example 3) in 30ml of dry dimethylformamide was then added during 10 minutes and the solution was stirred at 70°C for 1 hour. 100ml of distilled water were added at room temperature and the reaction mixture was poured into 400ml water. The mixture was extracted with diethyl ether (3x150ml). The extracts were washed with 200ml 1 N hydrochloric acid, 200ml water (twice),

100ml brine, dried over magnesium sulphate and evaporated to dryness to give the desired enantiomer of 2-[5-(3,5-dimethylpyrazol-1-yl)pentylsulphinyl]-4,5-diphenyl-1-[2-(trimethylsilyl)ethoxymethyl]imidazole (20.0g) in the form of a yellow oil which was used in Example 5 without further purification.
[NMR ($CDCl_3$):- 0.00 (s,9H); 0.89 (dd,2H); 1.50-2.05 (m,6H); 2.24 (s,6H); 3.40-3.71 (dm,2H); 3.50 (dd,2H); 4.02 (t,2H); 5.46 (dd,2H); 5.80 (s,1H); 7.20-7.55 (m,10H)].

EXAMPLE 5

A solution of 14.91g of crude 2-[5-(3,5-dimethylpyrazol-1-yl)pentylsulphinyl]-4,5-diphenyl-1-[2-(trimethylsilyl)ethoxymethyl]imidazole (as obtained in Example 4) in 250ml of ethanol was treated with 265ml of 1 N hydrochloric acid and the mixture was stirred at 50°C for 90 minutes. The solution was then neutralised to pH 7 at room temperature by treatment with 1N aqueous sodium hydroxide solution and extracted with diethyl ether (3x200ml). The combined extracts were washed with 100ml water, dried over magnesium sulphate and evaporated to dryness. The residue was subjected to flash chromatography on silica gel, eluting with a mixture of methanol and ethyl acetate, 5:95, to give 8.5g of a clear glass. This was treated with 80ml diethyl ether and the solution was left for 3 days at 2 to 5°C. A white solid was filtered off and the filtrate was concentrated to dryness, to give 6.52g of enantiomer A of 2-[5-(3,5-dimethylpyrazol-1-yl)pentylsulphinyl]-4,5-diphenylimidazole in the form of a white crisp solid.
[Enantiomeric excess: 98%, determined by chiral HPLC (column: Chiralcel OD; mobile phase: methanol:heptane: isopropanol 5:93:2; UV detection at 270nm).
$[\alpha]_D^{22}$ = +20.6° (c 0.97; dichloromethane)
$[\alpha]_D^{22}$ = -15.6° (c 1.02; methanol)
NMR ($CDCl_3$):- 1.37-1.95 (m,6H); 2.19 (s,3H); 2.21 (s,3H); 3.15-3.30 (m,2H); 3.96 (t,2H); 5.79 (s,1H); 7.28-7.54 (m,10H)].

EXAMPLE 6

Racemic 2-[5-(3,5-dimethylpyrazol-1-yl)pentyl)sulphinyl]-4,5-diphenylimidazole (500mg; prepared as described in Example 1) was subjected to preparative HPLC using a column (length 25cm, diameter 2cm) of cellulose tris(3,5-dimethylphenylcarbamate) on a silica gel support (as commercialised under the name "Chiralcel OD"), eluting with a mixture of methanol, isopropanol and n-heptane, in a ratio of 5:2:93v/v. There was thereby prepared enantiomer A of 2-[5-(3,5-dimethylpyrazol-1-yl)pentylsulphinyl]-4,5-diphenylimidazole, virtually free from enantiomer B (enantiomeric excess = 95%).

EXAMPLE 7

A mixture of the required enantiomer of 2-[5-(3,5-dimethylpyrazol-1-yl)pentylsulphinyl]-4,5-diphenyl-1-(4-methoxybenzyl)imidazole (32.4g; prepared as described in Reference Example 8) and anisole (25.4g) in trifluoroacetic acid (324ml) was stirred at reflux for 6 hours. The solvents were removed under reduced pressure and the residue was treated carefully with ice (150g). The pH of the solution was brought to 7-8 by treatment with saturated aqueous sodium bicarbonate solution. The mixture was then extracted with ethyl acetate (2x250ml) and the combined extracts were washed with water (250ml), dried over magnesium sulphate, and evaporated to dryness. The residue was subjected to flash chromatography on silica gel, eluting with a mixture of methanol and ethyl acetate (3:97v/v), to give a clear glass (21.8g). This was treated with t-butyl methyl ether (120ml) and the mixture was heated at reflux for 5 minutes and then allowed to cool to room temperature. The resulting white solid was filtered off and washed with t-butyl methyl ether (40ml), to give enantiomer A of 2-[5-(3,5-dimethylpyrazol-1-yl)pentylsulphinyl]-4,5-diphenylimidazole (20.10g) in the form of a white solid (m.p. 110-111°C, enantiomeric excess = 97%, determined by chiral HPLC (column: Chiralcel OD; mobile phase: methanol:heptane:isopropanol 5:93:2v/v; UV detection at 270nm).

This solid was recrystallised from a boiling mixture of t-butyl methyl ether (100ml) and methanol (6ml), to give enantiomer A of 2-[5-(3,5-dimethylpyrazol-1-yl)pentylsulphinyl]-4,5-diphenylimidazole (16.9g) in the form of a white solid (m.p. 112-114°C), enantiomeric excess = 99% [NMR($CDCl_3$]:- 1.37-1.95 (m,6H);2.19(s,3H);2.21 (s,3H);3.15-3.30 (m,2H);3.96 (t,2H);5.79(s,1H);7.28-7.54(m,10H)].

EXAMPLE 8

A mixture of 2-[5-(3,5-dimethylpyrazol-1-yl)pentylthio]-4,5-diphenylimidazole (2.08g; prepared as described in Reference Example 1) and so-called "(+)-8,8-dichlorocamphorylsulfonyloxaziridine" [1.64g; an item of

commerce sold by FLUKA; Davis et al, J. Org. Chem., (1990), 55, 3715-3717] in toluene (50ml) was stirred at 40°C for 3 hours. The mixture was then subjected to flash chromatography on silica gel, eluting with a mixture of methanol and ethyl acetate (5:95v/v), to give enantiomer A of 2-[5-(3,5-dimethylpyrazol-1-yl)pentylsulphinyl]-4,5-diphenylimidazole (2.2g) enantiomeric excess = 87%, determined by chiral HPLC (column: Chiralcel OD; mobile phase: methanol: heptane:isopropanol 5:93:2v/v; UV detection at 270nm).

This material was recrystallised twice from a mixture of t-butyl methyl ether and methanol (10:1v/v), to give enantiomer A of 2-[5-(3,5-dimethylpyrazol-1-yl)pentylsulphinyl]-4,5-diphenylimidazole (1.25g) enantiomeric excess = 98% [NMR($CDCl_3$):- 1.37-1.95 (m,6H);2.19(s,3H);2.21(s,3H);3.15-3.30 (m,2H);3.96 (t,2H);5.79 (s,1H);7.28-7.54(m,10H)].

EXAMPLE 9

Racemic 2-[5-(3,5-dimethylpyrazol-1-yl)pentylsulphinyl]-4,5-diphenyl-[2-(trimethylsilyl)ethoxymethyl]imidazole (5g; prepared by the reaction of racemic 2-[5-(3,5-dimethylpyrazol-1-yl)pentylsulphinyl]-4,5-diphenylimidazole with sodium hydride followed by 2-(trimethylsilyl)ethoxymethyl chloride, in dimethylformamide, at room temperature) was subjected to preparative HPLC using a column (length 25cm, diameter 2cm) of cellulose tris(3,5-dimethylphenylcarbamate) on a silica gel support (as commercialised under the name "Chiralcel OD"), eluting with a mixture of isopropanol and n-heptane, in a ratio of 10:90v/v. There was thereby prepared the desired enantiomer of 2-[5-(3,5-dimethylpyrazol-1-yl)pentylsulphinyl]-4,5-diphenyl-[2-(trimethylsilyl)ethoxymethyl]imidazole.

This material was converted, by proceeding as described in Example 5, to enantiomer A of 2-[5-(3,5-dimethylpyrazol-1-yl)pentylsulphinyl]-4,5-diphenylimidazole, free or virtually free from enantiomer B.

REFERENCE EXAMPLE 1

A stirred solution of 3,5-dimethylpyrazole (9.6g) in anhydrous dimethylformamide (100ml), cooled in an ice-bath, was treated with potassium tert-butoxide (11.2g). The ice-bath was removed and the mixture was stirred for one hour, the temperature rising to room temperature. The mixture was then treated with 1-bromo-5-chloropentane (18.6g), and the mixture was stirred at room temperature overnight, to give "reaction mixture 1".

A mixture of 4,5-diphenylimidazole-2-thiol (25.2g) and potassium tert-butoxide (11.2g) in anhydrous dimethylformamide (200ml) was stirred at room temperature for 30 minutes. This mixture was treated with "reaction mixture 1", and stirred at room temperature overnight. The mixture was filtered and the filtrate was evaporated to low bulk, and treated with dichloromethane (400ml) and water (200ml). The organic layer was washed with water (200ml), dried over magnesium sulphate, and evaporated. The resulting residue was triturated with diethyl ether (250ml), and filtered. The filtrate was evaporated and the resulting residue was subjected to flash chromatography on a silica gel column, using a mixture of ethyl acetate and dichloromethane (1:1v/v) as eluent. The resulting product was triturated with pentane, to give 2-[5-(3,5-dimethylpyrazol-1-yl)pentylthio]-4,5-diphenylimidazole (16.9g), in the form of a white solid, m.p. 75°C. [Elemental analysis:-C,71.9;H,6.78;N,13.5;S,8.0%; calculated:- C,72.1; H,6.73;N,13.5;S,7.69%].

REFERENCE EXAMPLE 2

To a solution of sodium hydride (5.38g of a 60% dispersion in mineral oil) in 450ml of dry dimethylformamide stirred at room temperature under nitrogen was added a suspension of 29.95g of 2-methylthio-4,5-diphenylimidazole (prepared as described by Haber et al., J.Med.Chem. 1985, 28, 1188-1194) in 180ml of dry dimethylformamide during 10 minutes. After 1 hour of stirring at room temperature, the mixture was cooled to 0°C and a solution of 20.89ml of 2-(trimethylsilyl)ethoxymethyl chloride in 180ml of dry dimethylformamide was added during 10 minutes and the solution was allowed to heat up to room temperature and was stirred for 2 hours. 250ml of distilled water were added and the reaction mixture was poured into 2000ml water and then it was extracted with diethyl ether (4x500ml). The combined extracts were washed with 400ml water (twice), 400ml brine, dried over magnesium sulphate and evaporated to dryness. The residue was subjected to flash chromatography on silica gel, eluting with a mixture of cyclohexane and ethyl acetate, 4:1, to give 40.28g of 2-methylthio-4,5-diphenyl-1-[2-(trimethylsilyl)ethoxymethyl]imidazole, in the form of a clear oil.
[NMR ($CDCl_3$):- 0.00 (s,9H); 0.90 (t,2H); 2.77 (s,3H); 3.48 (t,2H); 5.14 (s,2H); 7.13-7.53 (m,10h)].

REFERENCE EXAMPLE 3

A solution of 2-methylthio-4,5-diphenylimidazole (28.2g) in dry N-methylpyrrolidinone (215ml) stirred under nitrogen was treated with sodium hydride (4.66g of a 60% dispersion in mineral oil) at a temperature maintained below 30°C. The mixture was stirred at room temperature for 1 hour, and then a solution of 18.25g of 4-methoxybenzyl chloride in 35ml N-methylpyrrolidinone was added dropwise over 5 minutes and the mixture was stirred for a further hour. 250ml of toluene and 775ml of water were successively added and the aqueous layer was separated. The aqueous layer was extracted twice with 185ml toluene. The combined extracts were washed twice with 185ml water, dried over magnesium sulphate and evaporated to dryness, to give a product which was triturated with 360ml pentane. The resulting solid was filtered off and washed with 36ml pentane to give a solid (37g). Recrystallisation from 130ml of boiling methanol gave 32.6g of 2-methylthio-4,5-diphenyl-1-(4-methoxybenzyl)imidazole as a white solid, m.p.113-114°C.

REFERENCE EXAMPLE 4

To a solution of 146.4g of 4,5-diphenyl-2-mercaptoimidazole in dry N-methylpyrrolidinone (1160ml) stirred under a nitrogen atmosphere was added 23.3g of a 60% sodium hydride dispersion in mineral oil at a temperature maintained below 35°C. After stirring at room temperature for 1 hour, 37ml of iodomethane was added dropwise over 5 minutes and the mixture was stirred at room temperature for 1 hour. 27.9g of a 60% sodium hydride dispersion in mineral oil were added portionwise over 30 minutes and the mixture was stirred for 2 hours at room temperature. The mixture was warmed to 30-35°C and a solution of 100g of 4-methoxybenzyl chloride in 200ml N-methylpyrrolidinone was added dropwise over 30 minutes. The mixture was stirred at room temperature for 16 hours. 200ml of water was slowly added and the reaction mixture was poured into 2 litres of cold water and extracted with toluene (3x800ml). The combined extracts were washed twice with 1 litre of water, dried over magnesium sulphate and evaporated to dryness to give a crude product which was triturated with 1500ml of cold pentane. The solid was filtered off and washed twice with 300ml pentane to give 200g of crude product. Recrystallisation from 600ml of boiling methanol gave 161.4g of 2-methylthio-4,5-diphenyl-1-(4-methoxybenzyl)imidazole in the form of a white solid, m.p. 113-114°C.

REFERENCE EXAMPLE 5

To a solution of 21.35g of diethyl D-tartrate in 300ml of dry dichloromethane stirred at room temperature under nitrogen was added 14.7g of titanium isopropoxide. After 15 minutes stirring, the mixture was cooled to between -20°C and -25°C and a solution of 20g of 2-methylthio-4,5-diphenyl-1-(4-methoxybenzyl)imidazole (obtained as described in Reference Example 3 or 4) in 50ml of dry dichloromethane was added. After stirring for 20 minutes, 22.8g of cumene hydroperoxide (80%) was added dropwise over 10 minutes at -20°C to -25°C and the solution was stirred under nitrogen for 24 hours at -18 to -22°C. 10ml of distilled water was added and the mixture was vigorously stirred for 1 hour at room temperature. The gel obtained was filtered and washed with dichloromethane. The combined filtrates were stirred with 2N aqueous sodium hydroxide solution, washed with brine, dried over magnesium sulphate and evaporated to dryness. Flash chromatography on silica gel, eluting with mixtures of cyclohexane and ethyl acetate (8:2 to 1:1v/v) gave 17.5g of the desired enantiomer of 2-methylsulphinyl-4,5-diphenyl-1-(4-methoxybenzyl)imidazole in the form of a white solid, m.p. 147-149°C. Enantiomeric excess: 97.7%, determined by chiral HPLC (column: Chiralcel OD; mobile phase:methanol:heptane: isopropanol 5:93:2v/v; UV detection at 270nm).
[NMR($CDCl_3$):- 3.17(s,3H);3.77(s,3H);5.29(dd,2H);6.70-7.50(m,14H).

REFERENCE EXAMPLE 6

To a solution of 85.57g of diethyl D-tartrate in 1200ml of dry dichloromethane stirred at room temperature under nitrogen was added 58.98g of titanium isopropoxide. After 15 minutes stirring, the mixture was cooled to -20°C to -25°C and a solution of 160g of 2-methylthio-4,5-diphenyl-1-(4-methoxybenzyl)-imidazole (obtained as described in Reference Example 3 or 4) in 300ml of dry dichloromethane was added over 2 minutes. After stirring for 15 minutes, 92ml of cumene hydroperoxide (80%) was added dropwise over 5 minutes at -20°C to -25°C and the solution was stirred under nitrogen for 9.5 hours at -18 to -22°C. 38ml of distilled water was added and the mixture was vigorously stirred for 1 hour at room temperature. 80g of diatomaceous earth was added to the gel and the mixture was stirred for 5 minutes, filtered and washed with dichloromethane. The combined filtrates were stirred for 1 hour with of 2N aqueous sodium hydroxide solution, washed with water, twice with 5%w/v aqueous solution of sodium metabisulphite, once with brine, dried over magnesium sulphate and evaporated to dryness to give 260g of a yellow oil. 1.4 litre of cyclohexane wad added and the mixture was heated at reflux. 145ml of ethyl acetate was added and the mixture was cooled to room temperature and left in a cold room for 16 hours. The resulting white crystals were, washed twice with 300ml cyclohexane and dried to constant weight to give 125g of the desired enantiomer of 2-methylsulphinyl-4,5-diphenyl-1-(4-methoxybenzyl)imidazole, m.p. 147-148°C. Enantiomeric excess = 97.2%, determined by chiral HPLC (column:Chiralcel OD; mobile phase: methanol:heptane:isopropanol 5:93:2v/v; UV detection at 270nm).

REFERENCE EXAMPLE 7

To a solution of 13g of the required enantiomer of 2-methylsulphinyl-4,5-diphenyl-1-(4-methoxybenzyl)imidazole, (as obtained in Reference Example 6) in 170ml of dry tetrahydrofuran stirred at -70°C under nitrogen were successively added 16ml of hexamethylphosphoramide and, over 10 minutes, 18ml of a 2M solution of lithium diisopropylamide in a heptane: THF:ethyl benzene mixture. The temperature was allowed to warm up to -20 to -25°C and a solution of 9.2g of 4-chloro-1-iodobutane in 30ml dry tetrahydrofuran was added over 5 minutes at -20 to -25°C. The solution was stirred for 15 minutes at -20°C and warmed up to room temperature over 1 hour. 85ml of a 5% w/v aqueous solution of ammonium chloride were added to the solution and the mixture was extracted 3 times with 85ml diethyl ether. The combined extracts were washed with 85ml of 2N hydrochloric acid (twice), 85ml water, 85ml brine, dried over magnesium sulphate and evaporated to dryness. Flash chromatography on silica gel, eluting with a mixture of cyclohexane and ethyl acetate, 7:3v/v) gave 15.2g of 2-(5-chloropentylsulphinyl)-4,5-diphenyl-1-(4-methoxybenzyl)imidazole as an oil.
[NMR($CDCl_3$):- 1.50-1.95(m,6H);3.30-3.60(m,2H);3.53 (t,2H);3.77(s,3H);5.29(dd,2H);6.70-7.50(m,14H).

REFERENCE EXAMPLE 8

To a suspension of sodium hydride (0.61g of a 60% dispersion in mineral oil) in 50ml of dry N-methylpyrrolidinone stirred under nitrogen was added a suspension of 1.81g of 3,5-dimethylpyrazole in 20ml of dry N-methylpyrrolidinone over 20 minutes at a temperature maintained below 30°C. After stirring at room temperature for 45 minutes, 0.44g of sodium iodide was added. A solution of 5.82g of 2-(5-chloropentylsulphinyl)-4,5-diphenyl-1-(4-methoxybenzyl)imidazole (as obtained in Reference Example 7) in 20ml of dry N-methylpyrrolidinone was then added over 10 minutes and the solution was stirred at 100°C for 2 hours. 100ml of distilled water was added at room temperature and the mixture was poured into 300ml water. The mixture was extracted with 3 times 200ml of diethyl ether. The extracts were washed with 200ml of water, 200ml of brine, dried over magnesium sulphate and evaporated to dryness. Flash chromatography on silica gel, eluting with ethyl acetate gave 5.61g of the desired enantiomer of 2-[5-(3,5-dimethylpyrazol-1-yl)pentylsulphinyl]-4,5-diphenyl-1-(4-methoxybenzyl)imidazole as a clear oil.

This oil was crystallised from boiling cyclohexane to give the desired enantiomer of 2-[5-(3,5-dimethylpyrazol-1-yl)pentylsulphinyl]-4,5-diphenyl-1-(4-methoxybenzyl)imidazole in the form of a white crystalline solid; m.p. 107-108°C.
[NMR($CDCl_3$)]:- 1.40-1.90(m,6H);2.20(s,3H);2.21(s,3H); 3.35-3.55(dm,2H);3.76(s,3H);3.95(t,2H);5.28(dd,2H); 5.78 (s,1H);6.70-7.50(m,14H).

The present invention also includes within its scope pharmaceutical formulations which comprise enantiomer A or a pharmaceutically acceptable salt thereof in association with a pharmaceutically acceptable carrier or coating. In clinical practice the compounds of the present invention may be administered parenterally, rectally or orally.

Solid compositions for oral administration include compressed tablets, pills, powders and granules. In such solid compositions, enantiomer A is admixed with at least one inert diluent such as starch, sucrose or lactose. The compositions may also comprise, as is normal practice, additional substances other than inert diluents, e.g. lubricating agents, such as magnesium stearate.

Liquid compositions for oral administration include pharmaceutically acceptable emulsions, solutions, suspensions, syrups and elixirs containing inert diluents commonly used in the art such as water and liquid paraffin. Besides inert diluents such compositions may comprise adjuvants, such as wetting and suspending agents, and sweetening, flavouring, perfuming and preserving agents. The compositions according to the invention for oral administration also include capsules of absorbable material such as gelatin, containing enantiomer A with or without the addition of diluents or excipients.

Compositions according to the invention for parenteral administration include sterile aqueous, aqueous-organic, and organic solutions, suspensions and emulsions. Examples of organic solvents or suspending media are propylene glycol, polyethylene glycol, vegetable oils such as olive oil and injectable organic esters such as ethyl oleate. The compositions may also contain adjuvants such as stabilising, preserving, wetting, emulsifying and dispersing agents. They may be sterilised, for example, by filtration through a bacteria-retaining filter, by incorporation in the compositions of sterilising agents, by irradiation or by heating. They may also be manufactured in the form of sterile solid compositions, which can be dissolved in sterile water or some other

sterile injectable medium immediately before use.

Solid compositions for rectal administration include suppositories formulated in accordance with known methods and containing enantiomer A or a pharmaceutically acceptable salt thereof.

The percentage of active ingredient in the compositions of the invention may be varied, it being necessary that it should constitute a proportion such that a suitable dosage shall be obtained. Obviously, several unit dosage forms may be administered at about the same time. The dose employed will be determined by the physician, and depends upon the desired therapeutic effect, the route of administration, the duration of the treatment and the condition of the patient. In the adult, the doses are generally from about 0.01 to about 100, preferably 0.1 to 70, more especially 0.5 to 10, mg/kg body weight per day by oral administration, and from about 0.001 to about 10, preferably 0.01 to 1, mg/kg body weight per day by intravenous administration.

The following Example illustrates a pharmaceutical composition according to the present invention.

## COMPOSITION EXAMPLE

No. 2 size gelatin capsules each containing:-

| | |
|---|---|
| enantiomer A | 20 mg |
| lactose | 100 mg |
| starch | 60 mg |
| dextrin | 40 mg |
| magnesium stearate | 1 mg |

were prepared in accordance with the usual procedure.

## Claims

1. The enantiomer of 2-[5-(3,5-dimethylpyrazol-1-yl)pentylsulphinyl]-4,5-diphenylimidazole which, in dilute solution in dichloromethane at room temperature, rotates plane-polarised light of the sodium D wavelength to the right, or a pharmaceutically acceptable acid addition salt thereof.

2. A process for the preparation of the enantiomer defined in claim 1 which comprises:-
   (A) the separation of the enantiomer from a mixture of enantiomers of 2-[5-(3,5-dimethylpyrazol-1-yl)pentylsulphinyl]-4,5-diphenylimidazole by chromatography using a chiral adsorbent;
   (B) the replacement by a hydrogen atom of the protective group Y of the corresponding enantiomer of a compound of the general formula VI

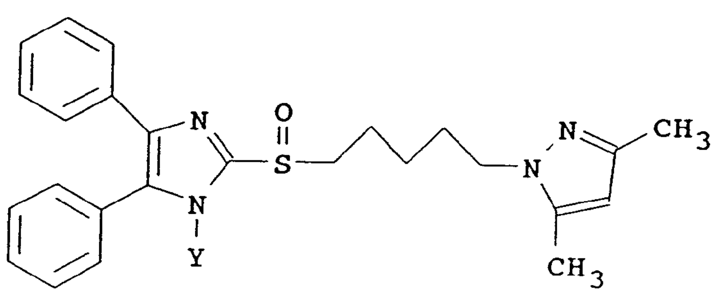

VI

   wherein Y represents a protective group;
   (C) the oxidation, using an enantio-selective oxidizing system, of 2-[5-(3,5-dimethylpyrazol-1-yl)pentylthio]-4,5-diphenylimidazole;
   optionally followed by the conversion of the enantiomer thus obtained into a pharmaceutically acceptable acid addition salt thereof.

3. A pharmaceutical composition which comprises the enantiomer defined in claim 1, or a pharmaceutically acceptable salt thereof, in association with a pharmaceutically acceptable carrier or coating.

4. A pharmaceutical composition useful in the treatment of a condition which can be ameliorated by administration of an inhibitor of acyl coenzyme-A:cholesterol-0-acyl transferase which comprises an amount effective to ameliorate said condition of the enantiomer defined in claim 1 or a pharmaceutically acceptable salt thereof.

5. A method for the treatment of a human or animal host suffering from, or subject to, a condition which can be ameliorated by administration of an inhibitor of acyl coenzyme-A:cholesterol-0-acyl transferase which comprises the administration to said host of an amount effective to ameliorate said condition of the enantiomer defined in claim 1 or a pharmaceutically acceptable salt thereof.

European Patent Office

# EUROPEAN SEARCH REPORT

Application Number

EP 92 30 6404

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| D,X | WO-A-9 110 662 (RHONE-POULENC RORER LIMITED) * line 20 - line 24 * | 1-5 | C07D403/12 A61K31/415 |
| A | PATENT ABSTRACTS OF JAPAN vol. 12, no. 40 (C-474)(2887) 5 February 1988 & JP-A-62 187 469 ( TAISHO PHARMACEUT CO LTD ) 15 August 1987 * abstract * | | |
| A | CHEMICAL ABSTRACTS, vol. 111, no. 11, 11 September 1989, Columbus, Ohio, US; abstract no. 97243C, TSUJI,MASAYOSHI; A.O.: 'Preparation of (heterocyclylalkylthio- or -sulfinyl)diphenylimidazoles as antiulcer and antiinflammatory agents' page 746 ;column 2 ; * abstract * & JP-A-0 140 467 (HISAMITSU PHARMACEUTICAL CO. INC.) | | |

TECHNICAL FIELDS SEARCHED (Int. Cl.5 )

C07D
A61K

The present search report has been drawn up for all claims

| Place of search | Date of completion of the search | Examiner |
|---|---|---|
| THE HAGUE | 28 AUGUST 1992 | DE BUYSER I.A.F. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)